# EUROPÄISCHE PATENTSCHRIFT

(11) **EP 2 361 585 B1**
(45) Veröffentlichungstag und Bekanntmachung des Hinweises auf die Patenterteilung: **06.09.2017**
(21) Anmeldenummer: 11155279.0
(22) Anmeldetag: 22.02.2011
(51) Int. Cl.: A61C 7/14, A61C 7/00, A61C 9/00, A61C 7/16

(54) **Bracketsystem und Verfahren zur Planung und Herstellung eines Bracketsystems zur Korrektur von Zahnfehlstellungen**
Bracket system and method for planning and producing a bracket system for correcting dental misalignments
Système de bracket et procédé de planification et de fabrication d'un système de bracket pour la correction de mauvaises positions de dents

(30) Priorität: 22.02.2010 DE 102010002206
(43) Veröffentlichungstag der Anmeldung: 31.08.2011
(73) Patentinhaber: Sirona Dental Systems GmbH, 64625 Bensheim (DE)
(72) Erfinder: Schiemann, Christian, 86356 Neusäß (DE)
(74) Vertreter: Sommer, Peter

(56) Entgegenhaltungen:
- US-A1- 2004 029 068
- US-A1- 2005 239 013

## Beschreibung

### Technisches Gebiet

Die Erfindung betrifft ein Bracketsystem und ein Verfahren zur Planung dieses Bracketsystems zur Korrektur von Fehlstellungen von Zähnen, bestehend aus mehreren Brackets und einem Bogen.

### Stand der Technik

Bei der Verwendung von bekannten Bracketsystemen tritt das Problem des fehlerhaften Beklebens auf. Dabei werden die Brackets nicht exakt an den idealen vorgesehenen Positionen auf den Patientenzähnen aufgeklebt, so dass dadurch das Behandlungsziel nicht in der gewünschten Weise erreicht werden kann.

Aus dem Stand der Technik sind mehrere Lösungen bekannt, um das fehlerhafte Bekleben zu verhindern, z.B. US2004/0029068 und US2005/0239013. Eine Möglichkeit ist das sogenannte indirekte Kleben der Brackets auf die Patientenzähne mit Übertragungstrays. Auf der Grundlage eines Abdrucks wird zunächst aus Gips ein Meistermodell hergestellt. Danach wird das Meistermodell dubliert und aus Gips ein Zahnkranz hergestellt. Der Zahnkranz wird aus einer Silikon-Dublierform entnommen und die einzelnen Zähne mit einer Diamantscheibe von apikal bis kurz vor dem Kontaktpunkt getrennt, die Stümpfe konisch abgeschliffen und mit flüssigem Wachs übergossen. Zusätzlich werden in den Gips senkrechte Rillen mittig entlang der Zähne verlaufend eingefräst, die eine eindeutige Positionierung gewährleisten. Duplikatmodelle beider Kiefer werden hergestellt und in einen Artikulator eingesetzt. Ein Zahntechniker formt dann nach den Anweisungen des behandelnden Zahnarztes unter Erwärmung des Wachses ein ideales Setupmodell, das dem Behandlungsziel entspricht. Anschließend erfolgt die Positionierung der Brackets. Die Brackets werden auf das Setupmodell geklebt. Dabei wird zunächst eine Modellposition gesucht, in der sich alle Brackets in einer Ebene platzieren lassen und weder die Gingiva noch die antagonistischen Zähne stören. Nachdem alle Brackets geklebt sind, werden Messelemente in die Schlitze an den Brackets eingesetzt und anschließend mit einer stereoskopischen Kamera in einer ersten Aufnahme und einer zweiten Aufnahme aus einem versetzten Winkel vermessen. Aus den beiden Aufnahmen wird eine dreidimensionale Darstellung des Setupmodells mit den Brackets und den Messelementen errechnet. Daraufhin werden die Brackets vorsichtig von den Zahnoberflächen gelöst und mit einem wasserlöslichen Kleber auf das Meistermodell mit einer Fehlstellung der Patientenzähne geklebt. Die senkrechten Rillen gewährleisten dabei eine eindeutige Positionierung. Nachdem alle Brackets samt ihrer Messelemente auf das Meistermodell übertragen wurden, werden weitere Aufnahmen mit einer stereoskopischen Kamera hergestellt und eine dreidimensionale Darstellung errechnet. Aus der dreidimensionalen Darstellung der Messelemente lässt sich die genaue Position der Brackets errechnen. Anschließend werden die Messelemente entfernt und so genannte Übertragungstrays für die Übertragung der Brackets auf die Patientenzähne hergestellt. Als Material für die Übertragungstrays wird oft ein transparentes Silikon verwendet. Nachdem die Übertragungstrays am Meistermodell ausgeformt und ausgewertet sind, werden sie abgenommen und zusammen mit den Brackets auf die Patientenzähne geklebt. Nach dem Austrocknen des Klebers werden die Übertragungstrays aus Silikon abgezogen.

Als eine Weiterbildung zur Verbesserung der Genauigkeit der Übertragung kann auch ein Kleberoboter (Orthorobot) verwendet werden, der die Brackets an den idealen von einer Software ermittelten Positionen am Setupmodell anbringt. Die Übertragung erfolgt jedoch weiterhin unter Verwendung der Übertragungstrays.

Der Nachteil dieses Verfahrens liegt darin, dass die Übertragung mittels Übertragungstrays durch den Zahnarzt manuell erfolgt und fehlerbehaftet ist. Die fehlerhafte Positionierung ist dadurch bedingt, dass die Übertragungstrays unzureichend durch den Zahntechniker an die Zahnoberfläche angepasst wurden, beim Abziehen vom Meistermodell verformt wurden oder beim Aufbringen auf die Patientenzähne durch den Zahnarzt fehlerhaft aufgebracht wurden.

Eine weitere bekannte Möglichkeit ist die so genannte "Incognito"-Positionierung der Brackets. Bei dieser Form der Behandlung handelt es sich um eine ästhetische, da von außen völlig unsichtbare, kieferorthopädische Zahnkorrektur. Die speziellen Brackets werden auf der Innenseite der Zähne lingual befestigt. Jedes einzelne Bracket wird in einem Labor speziell für den Patienten angepasst. Die Herstellung erfolgt meist mittels einer computergesteuerten CAD/CAM-Technologie. Insbesondere werden die Brackets mit genau passenden lingualen Flächen für den jeweiligen Patienten gefertigt.

Die Positionierung der Brackets erfolgt meist indirekt unter Verwendung von Übertragungstrays. Bei Reparaturen werden die Brackets in der Regel auch direkt durch freies Kleben auf den Patientenzähnen angebracht.

Der Nachteil dieses Verfahrens liegt darin, dass die Positionierung mittels Übertragungstrays mit den oben genannten Problemen verbunden.

Eine weitere bekannte Möglichkeit der Positionierung ist freies Kleben durch den Zahnarzt nach vorheriger Markierung des Abstandes des Brackets zur Spitze des jeweiligen Zahns. Der Nachteil dieses Verfahrens ist dass, nur der Abstand zur Zahnspitze eingehalten werden kann und damit keine genaue dreidimensionale Positionierung bezüglich der Tiefe, des seitlichen Abstands und des relativen Winkels zwischen der Ausrichtung des Brackets und des jeweiligen Zahns möglich ist.

Die Aufgabe dieser Erfindung besteht daher darin, eine genauere Positionierung der Brackets auf den Patientenzähnen zu ermöglichen, um das geplante Behandlungsziel, nämlich die vollständige Korrektur der Fehlstellungen der Patientenzähne zu erreichen.

### Darstellung der Erfindung

Die Erfindung ist in den Ansprüchen 1 und 10 definiert. Ein Erfindungsgegenstand ist ein Verfahren zur Planung eines Bracketsystems zur Korrektur von Fehlstellungen von Zähnen, bestehend aus mehreren Brackets und einem Bogen. Auf bestimmten Zahnoberflächen der zu korrigierenden Zähne wird pro Zahn jeweils mindestens ein Plättchen angebracht. Anschließend wird eine dreidimensionale optische Aufnahme des zu behandelnden Zahnbereichs aufgenommen, wobei die Plättchen Registrierungselemente mit Registrierungspunkten aufweisen, die eine charakteristische Form zur Registrierung in der dreidimensionalen optischen Aufnahme aufweisen. Das Bracketsystem besteht aus mehreren Brackets, einem Bogen und mehreren Plättchen. Im Unterschied zu bekannten Brackets werden zuerst die Plättchen auf die jeweiligen Zahnoberflächen, die beispielsweise labial oder lingual angeordnet sein können, geklebt, deren Positionen in einer dreidimensionalen optischen Aufnahme und einer dreidimensionalen Röntgenaufnahme vermessen, anschließend bekannte handelsübliche Brackets mit einer Aussparung auf einer Klebefläche der Brackets versehen, die der Form der Plättchen entspricht. Im nächsten Schritt werden die präparierten Brackets auf der Klebefläche mit einer Klebeschicht versehen und auf die Plättchen aufgesetzt und mit der Zahnoberfläche verklebt. Dadurch wird eine im Vergleich zum bisherigen Verfahren genauere Positionierung der Brackets auf den Patientenzähnen erreicht.

Die Brackets bilden einen Ansatzpunkt zur mechanischen Bewegung der Zähne bei festsitzenden Apparaturen. Ein Bracket ist eine Vorrichtung für die Aufnahme des Bogens mittels eines Schlosses, der als ein horizontal verlaufenden Schlitz ausgebildet ist. Die Brackets werden unter Verwendung von Ligaturen oder Pins am Bogen befestigt. Falls die Brackets auf der Außenfläche der Zähne befestigt sind, spricht man von bukkalen beziehungsweise labialen Brackets und falls Sie auf der inneren Fläche der Zähne angeordnet sind, spricht man von lingualen Brackets. Die Brackets können aus rostfreiem Edelstahl, Gold, Keramik, Composit oder Titan gefertigt sein. Bei vielen Anwendungen müssen die Brackets über den Bogen gleiten. Dabei können die Reibungskräfte mittels der Ligaturen geändert werden. Der Bogen verläuft um den gesamten Zahnbogen. Die Korrektur der Zähne erfolgt mittels einer kleinen Kraft auf die Zähne, die vom elastischen Bogen ausgeht und mittels der geklebten Brackets auf die Patientenzähne übertragen wird.

Eine dreidimensionale optische Aufnahme kann mittels einer intraoralen Dentalkamera erfolgen, die beispielsweise mittels eines Triangulationsverfahrens eine dreidimensionale optische Aufnahme der zu behandelnden Patientenzähne erstellt.

Die Plättchen weisen Registrierungselemente mit Registrierungspunkten auf, die eine charakteristische Form zur Registrierung in der dreidimensionalen optischen Aufnahme haben. Die charakteristische Form kann beliebig gestaltet sein, beispielsweise in Form von Erhebungen oder Aussparungen.

Ein Vorteil des erfinderischen Verfahrens ist, dass eine bessere Positionierung der Brackets auf den Patientenzähnen erreicht wird als bei den oben dargestellten herkömmlichen Verfahren, denn der Benutzer muss die präparierten Brackets nur noch auf die geklebten Plättchen aufsetzen und kann dabei keine Positionierungsfehler begehen. Durch die bessere Positionierung der Brackets wird folglich auch die Wahrscheinlichkeit eines erfolgreichen Behandlungsziels verbessert.

Ein Vorteil des erfinderischen Verfahrens ist, dass ein bereits bekanntes Bracketsystem beibehalten werden kann, da nach dem erfinderischen Verfahren beliebige handelsübliche Brackets präpariert werden können.

Vorteilhafterweise kann zusätzlich zur dreidimensionalen optischen Aufnahme eine dreidimensionale Röntgenaufnahme des zu behandelnden Zahnbereichs aufgenommen werden, wobei die Registrierungselemente mit Registrierungspunkten der Plättchen derart aufgebaut sind, dass sie zur Registrierung in der dreidimensionalen Röntgenaufnahme bezüglich Röntgenstrahlung sensitiv sind, indem die Registrierungspunkte aus einem Material aufgebaut sind, der einen Absorptionskoeffizienten bezüglich Röntgenstrahlung aufweist, der sich deutlich vom Absorptionskoeffizienten des übrigen Materials der Plättchen unterscheidet.

Die dreidimensionale Röntgenaufnahme des Oberkiefers und des Unterkiefers kann beispielsweise mittels der Computertomographie (CT) oder der so genannten Digitalen-Volumen-Tomographie (DVT) erstellt werden. Bei der DVT werden im Gegensatz zur CT zur Berechnung dreidimensionaler Strukturen zweidimensionale Bilder als Datensatz erzeugt, während bei einer CT die Bildgebung auf einer eindimensionalen Detektion beruht. Ein digitaler Volumen-Tomograph besteht aus einer rotierenden Röntgenquelle und einem CCD-Sensor, die um 180° oder 360° um den fixierten Patienten rotiert werden. Dabei werden während der Drehung mehrere zweidimensionale Summations-Einzelbilder erstellt und aus den gewonnenen Einzelbildern ein dreidimensionales Modell errechnet. Daraus können Schnittbilder in allen Raumebenen sowie dreidimensionale Ansichten berechnet werden.

Vorteilhafterweise werden mittels einer Planungseinheit zur Planung des Bracketsystems computergestützt die Bilddaten der dreidimensionalen optischen Aufnahme und die Bilddaten der dreidimensionalen Röntgenaufnahme durch Überlagerung von Registrierungspunkten der Registrierungselemente in der dreidimensionalen optischen Aufnahme und der dreidimensionalen Röntgenaufnahme zusammengeführt, so dass die eindeutige Lagebeziehung der Plättchen zur optischen dreidimensionalen Aufnahmen und zur dreidimensionalen Röntgenaufnahme festgestellt wird.

Das Auffinden der Registrierungspunkte in der dreidimensionalen optischen Aufnahme und der dreidimensionalen Röntgenaufnahme kann mittels eines Matching-Verfahrens oder mittels einer Mustererkennung erfolgen, wobei die Aufnahme nach einem Suchmuster der Registrierungselemente abgesucht wird. Die Identifizierung der Registrierungssegmente kann auch visuell mittels der Planungseinheit durch einen Benutzer erfolgen. Die Planungseinheit kann ein herkömmlicher Computer mit einem Bildschirm, Maus und Tastatur sein auf dem eine entsprechende Software zur Verarbeitung der Bilddaten installiert ist.

Vorteilhafterweise erfolgt mittels der Planungseinheit eine computergestützte Planung des anzufertigenden Bracketsystems anhand der überlagerten dreidimensionalen optischen Aufnahme und der dreidimensionalen Röntgenaufnahme.

Bei der Planung des Bracketsystems wird insbesondere die Lage der Brackets auf den Patientenzähnen und der Verlauf des elastischen Bogens bestimmt, so dass die korrigierenden Kräfte, die durch Torsionen oder Biegungen des Bogens verursacht werden, den Fehlstellungen der Patientenzähne entgegenwirken.

Vorteilhafterweise wird anhand der dreidimensionalen optischen Aufnahme ein virtuelles Meistermodell des Oberkiefers und/oder Unterkiefers erstellt, das die Fehlstellungen der Zähne des Patienten beinhaltet. Durch den Benutzer werden mittels der Planungseinheit die einzelnen Zähne virtuell korrigiert und ein ideales virtuelles Setupmodell wird erstellt, bei dem alle Fehlstellungen der Zähne des Patienten korrigiert sind. Aus den Abweichungen des Meistermodells und des Setupmodells werden korrigierende Positionsänderungen der einzelnen Zähne berechnet, wobei aus den korrigierenden Positionsänderungen die Anordnung der Brackets und des Bogens des Bracketsystems bestimmt wird.

Der Vorteil dieser Weiterbildung ist, dass die bekannten Verfahrensschritte des Verfahrens zum Bekleben der Brackets auf die Patientenzähne mit Übertragungstrays virtuell computergestützt durchgeführt wird, nämlich die Erstellung eines Meistermodells und eines korrigierten Setupmodells. Aus den Korrekturen des Benutzers können dann die notwendige Positionsänderung jedes einzelnen Zahns computergestützt automatisch berechnet werden und in Kenntnis der genauen Abmessungen der Brackets und des Bogens sowie deren mechanischer Eigenschaften automatisch eine Planung durchgeführt werden.

Ein weiterer Vorteil dieser Weiterbildung ist, dass ein Meistermodell und ein Setupmodell nicht mehr aufwändig im Labor hergestellt werden müssen und die Planung mittels der Planungseinheit unmittelbar nach der Vermessung in der Praxis eines Zahnarztes durchgeführt werden kann und anschließend mittels einer CAD/CAM-Einheit die Brackets präpariert werden können. Damit wird die Behandlungsdauer deutlich verkürzt und durch die Automatisierung im Gegensatz zur herkömmlichen Herstellung der Modelle im Labor die Genauigkeit verbessert.

Vorteilhafterweise können bei der Planung des Bracketsystems Faktoren aus der optischen dreidimensionalen Aufnahmen, nämlich die räumliche Anordnung und Form der Zähne, und weitere Faktoren aus der dreidimensionalen Röntgenaufnahme über eine Verteilung von harten und weichen Gewebe, nämlich die Position und die Abmessungen von Zahnwurzeln innerhalb einer apikalen Basis eines Kiefers des Patienten, die Abmessungen des Kieferknochens, anatomische Grenzen, Form der Gingiva, Nasenboden und/oder die Position des Gaumens, berücksichtigt werden. Dabei kann aus der Anordnung des harten und weichen Gewebes unter Heranziehung der bekannten Materialkonstanten der verschiedenen Gewebearten eine Erfolgswahrscheinlichkeit der gewünschten mechanischen dauerhaften Positionsänderung der Zähne nach dem Setupmodell berechnet werden.

In Kenntnis der mechanischen Eigenschaften der verschiedenen Gewebearten sowie der Brackets und des Bogens, wie das Elastizitätsmodul, Torsionsmodul, Druckfestigkeit, Zugfestigkeit und die Biegefestigkeit, kann computergestützt durch eine numerische Simulation, wie die so genannte Mehrkörpersimulation, die Erfolgswahrscheinlichkeit für die Positionsänderungen der einzelnen Zähne berechnet werden. Bei der Mehrkörpersimulation werden reale Körper durch mehrere unverformbare Körper abgebildet. Dabei kann insbesondere bei Kindern die Wachstumsrichtung der skelettalen Basen des Gesichtsschädels und der Zähne berücksichtigt werden. Die Reaktionen des Kieferknochens beziehungsweise des Gewebes auf die korrigierenden Kräfte ist anisotrop, so dass die Erfolgswahrscheinlichkeit numerisch computergestützt berechnet werden kann.

Vorteilhafterweise können mittels der Planungseinheit alternative Empfehlungen zur Behandlung, wie Extraktion, Strippen oder Operieren, vorgeschlagen werden, falls die von der Planungseinheit berechnete Erfolgswahrscheinlichkeit gering ist.

Falls die berechnete Erfolgswahrscheinlichkeit zu gering ist, zeigt die Planungseinheit an, dass die Behandlung voraussichtlich nicht zu dem gewünschten Behandlungserfolg führen wird und schlägt alternative Behandlungen vor. Bei der Extraktion handelt es sich um die Entfernung eines Zahns ohne operative Eingriffe. Beim Strippen (approximale Schmelzreduktion) werden die Seiten der Patientenzähne leicht beschliffen, um Platz zu den Nachbarzähnen zu gewinnen. Bei operativen Eingriffen werden die skelettale Einheiten (Maxilla, Mandibula, Alveolarfortsätze) entsprechend ihrer Fehlstellung zur Erzielung einer optimalen Lagebeziehung chirurgisch korrigiert.

Vorteilhafterweise können zusätzlich durch den Benutzer individuelle Behandlungsstrategien, nämlich ein beliebiges Okklusionskonzept(z.B. nach Angle, nach Andresen oder eine bioästhetische Versorgung), eine Berücksichtigung der Vorgeschichte des jeweiligen Patienten, voraussehbare Wachstumsentwicklung und/oder eine notwendige Überkompensation der Korrektur eingegeben werden und bei der Planung des Bracketsystems berücksichtigt werden.

Zur Verbesserung des Behandlungsziels können durch den Benutzer individuelle Behandlungsstrategien eingegeben werden und werden bei der Planung berücksichtigt, indem eine Abwägung zwischen den verschiedenen Faktoren bezüglich mechanischer Stabilität der idealen Ausrichtung der Zähne nach dem Setupmodell und den eingegeben Behandlungsstrategien durchgeführt wird. Die meisten Okklusionskonzepte, wie eine bioästhetische Versorgung, lassen die unteren bukkalen Höckerspitzen in die antagonistischen Gruben in den Oberkieferzähnen okkludieren. Beim Aufstellen des digitalen Setups muss der Benutzer die entsprechende Punkte, beispielsweise den Punkt des mesialen Höckers von Zahn 46, nach dem Zahnschema, markieren und beispielsweise zum Punkt an der Randleiste des Zahns 16 führen. Eine Überkompensation der Korrektur ist vor allem bei einem stark rotierten Zahn erforderlich, der beispielsweise mehr als um 35° derotiert wird. Dies ist erforderlich, da nach dem Entfernen des Bracketssystems und der erfolgten Korrektur der Zahn sich um etwa 5° bis 10° wieder zurückdreht.

Vorteilhafterweise kann jedes Plättchen Registrierungselemente mit mindestens drei Registrierungspunkten aufweisen. Dadurch wird die räumliche Orientierung der Plättchen in der dreidimensionalen optischen Aufnahme und der dreidimensionalen Röntgenaufnahme ermöglicht. Dabei können die drei Registrierungspunkte in einem beispielsweise gleichseitigen Dreieck angeordnet sein. Erfindungsgemäß werden nach der Planung des Bracketsystems mittels der Planungseinheit vorgefertigte Brackets mittels einer CAD/CAM-Einheit an die aufgeklebten Plättchen angepasst, indem auf einer Klebefläche jedes Brackets eine Aussparung herausgearbeitet wird, die als Gegenstück der Form des Plättchens entspricht, so dass beim Befestigen des jeweiligen Brackets an dem entsprechenden Plättchen die geplante Position relativ zum Zahn erreicht wird.

Dadurch wird eine zeitsparende automatisierte Präparation der Brackets ermöglicht, die weniger fehleranfällig ist als die manuelle Anpassung durch einen Zahnlaboranten. Die Position der Aussparung wird mittels der Planungseinheit bestimmt, indem die geplante Position des Brackets auf dem Zahn zur Position des aufgeklebten Plättchens in Relation gesetzt wird.

Erfindungsgemäß werden die vorgefertigten Brackets mittels einer CAD/CAM-Einheit an das aufgeklebte Plättchen angepasst und die gesamte Klebefläche jedes Brackets wird als Gegenstück entsprechend der Form der jeweiligen Zahnoberfläche geformt, um eine einwandfreie Passung zu ermöglichen.

Zusätzlich zur Fertigung einer Aussparung wird die gesamte Klebefläche so bearbeitet, dass sie zu der zu beklebenden Zahnoberfläche passt. Dies ermöglicht eine bessere Positionierung des Brackets auf dem Zahn, da das Bracket exakt auf der Zahnoberfläche aufliegt und Verkippungen, die zu einer fehlerhaften Positionierung führen könnten, ausgeschlossen werden.

Die Klebefläche kann auch mit einer Kunststoffschicht beschichtet werden und nur diese Kunststoffschicht zur Anpassung an die zu beklebende Zahnoberfläche bearbeitet werden. Dadurch wird die Nutzungsdauer des Werkzeugs im Vergleich zur Bearbeitung von Metall erhöht.

Vorteilhafterweise können nach dem Anpassen der Brackets an die jeweiligen Zahnoberfläche die Brackets manuell auf die jeweiligen Zahnoberfläche mittels eines Klebers geklebt werden.

Als Kleber kann ein spezieller Zwei-Komponenten-Kleber verwendet werden, der lichtpolymerisierend ist. Beim Kleben ist darauf zu achten, dass in der Klebeschicht keine Luftblasen entstehen sowie der gesamte Zwischenraum zwischen der Klebefläche des Brackets und der Zahnoberfläche mit der Kleberschicht ausgefüllt ist.

Vorteilhafterweise können die zu beklebenden Zahnoberflächen labiale Zahnoberflächen und/oder linguale Zahnoberflächen sein.

Abhängig davon, ob das Bracketsystemen auf der äußeren Seite der Zähne oder auf der inneren Seite der Zähne verläuft, werden die passenden buckalen bzw. lingualen Brackets verwendet.

Die einzelnen Schritte des erfinderischen Verfahrens können alle vom Zahnarzt selbst in der Praxis unter Verwendung der Planungseinheit mit einer CAD/CAM-Einheit durchgeführt werden. Das Bekleben der Zähne mit Plättchen vor der Registrierung kann auch durch eine Zahnarzthelferin durchgeführt werden. Das Erstellen des virtuellen Setupmodells mit speziellen Parametern, die vom Zahnarzt vorgegeben wurden, kann auch durch einen Zahntechniker erstellt werden. Erst das Einsetzen und das Kleben der präparierten Brackets auf die Plättchen sollte durch einen Zahnarzt durchgeführt werden.

Ein weiterer Gegenstand der Erfindung ist ein Bracketsytem zur Korrektur von Fehlstellungen, von Zähnen, bestehend aus mehreren Brackets, einem Bogen und mehreren Plättchen. Die Plättchen weisen eine Klebefläche zum Kleben auf eine bestimmte Zahnoberfläche eines zu korrigierenden Zahns und Registrierungselemente mit Registrierungspunkten auf, die eine charakteristische Form zur Registrierung in einer dreidimensionalen optischen Aufnahme ermöglichen.

Das erfinderische Bracketsystem ist zur Durchführung des Verfahrens nach Anspruch 1 vorgesehen. Die Plättchen weisen Registrierungselemente mit Begünstigungspunkten auf, wobei die charakteristische Form als eine Erhebung oder eine Aussparung ausgestaltet sein kann, um das Auffinden in der optischen dreidimensionalen Aufnahmen zu ermöglichen.

Vorteilhafterweise können die Registrierungselemente mit Registrierungspunkten bezüglich Röntgenstrahlung sensitiv sein, wobei die Registrierungspunkte aus einem Material aufgebaut sind, der einen Absorptionskoeffizienten bezüglich Röntgenstrahlung aufweist, der sich deutlich vom Absorptionskoeffizienten des übrigen Materials der Plättchen unterscheidet, um die Registrierung in einer dreidimensionalen Röntgenaufnahme zu ermöglichen.

Dadurch werden die Registrierungspunkte auf der dreidimensionalen Röntgenaufnahme sichtbar und können mit der optischen dreidimensionalen Aufnahmen der Registrierungspunkte überlagert werden.

Vorteilhafterweise können die Brackets eine Aussparung aufweisen, die als Gegenstück zu der Form der Plättchen geformt ist, um eine genaue Passung des Plättchens an das jeweilige Bracket zu gewährleisten.

Dadurch kann das Bracket auf das passende Plättchen gesteckt werden, so dass eine sehr genaue Positionierung des Brackets auf dem Zahn ermöglicht wird.

Vorteilhafterweise können die Brackets eine Klebefläche aufweisen, die jeweils als Gegenstück zu der zu beklebenden Zahnoberfläche der Zähne geformt ist, um eine bessere Passung der Klebung zu erreichen.

Dadurch wird die Positionierung des Brackets auf dem Zahn verbessert, da das Bracket exakt auf Vertagung gleichauf liegt, da das Bracket exakt auf der Zahnoberfläche auffliegt.

### Kurzbeschreibung der Zeichnungen

Ausführungsbeispiele der Erfindung sind in der Zeichnung dargestellt. Es zeigt die
- Fig. 1: eine Skizze eines Bracketssystems;
- Fig. 2a: eine Skizze einer ingualen Zahnoberfläche mit einem Plättchen;
- Fig. 2b: eine Skizze des Schneidezahns aus Fig. 2a in einem weiteren Schritt des erfinderischen Verfahrens;
- Fig. 3: eine Ausführungsform der Planungseinheit;
- Fig. 4: eine Skizze eines Meistermodells;
- Fig. 5: eine Skizze des virtuelle Meistermodells aus Fig. 3 und Fig. 4 mit Fehlstellungen von Patientenzähnen;
- Fig. 6: eine Skizze eines korrigierten idealen Setupmodells;
- Fig. 7: zeigt eine Skizze einer CAD/CAM-Einheit zur Anpassung eines nicht präparierten Brackets;
- Fig. 8A: zeigt eine Skizze eines alternativen nicht präparierten Brackets vor der Bearbeitung;
- Fig. 8B: zeigt eine Skizze eines alternativen nicht präparierten Brackets nach der Bearbeitung.

### Ausführungsbeispiele

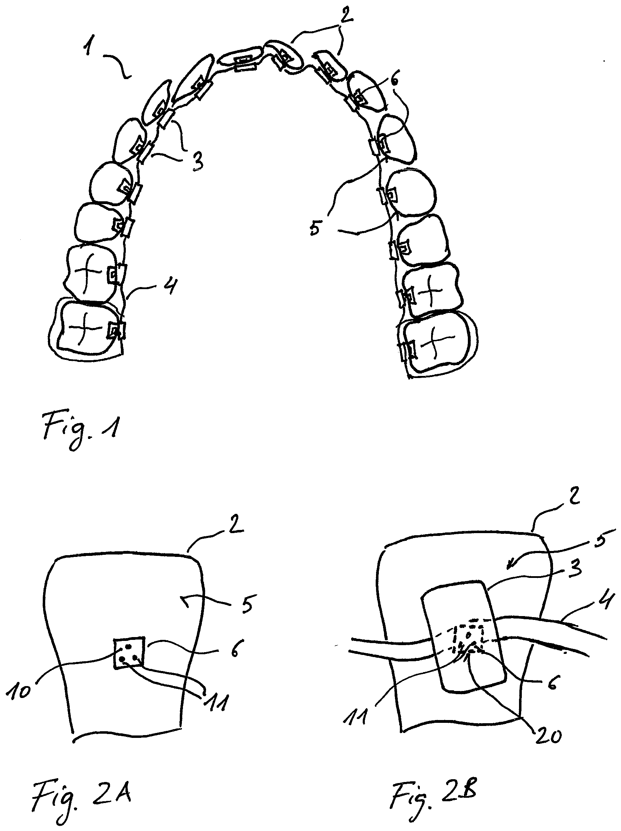

Die Fig. 1 zeigt eine Skizze des Bracketssystems 1 zur Korrektur von Fehlstellungen der Patientenzähne 2. Dabei besteht das Bracketsystem 1 aus mehreren Brackets 3 und einem Bogen 4, der entlang der Innenfläche der Zahnreihe verläuft. Eine Ausführungsform des Bracketssystems für die labiale bzw. bukkale Seite der Patientenzähne 2 ist ebenfalls möglich, wobei jedoch spezielle bukkale Brackets verwendet werden. Auf den inneren lingualen Zahnflächen der zu korrigierenden Patientenzähne 2 sind Plättchen angebracht, die zur Positionierung der Brackets 3 dienen. Das Verfahren der Positionierung wird im Folgenden detaillierter beschrieben.

Das Grundprinzip lässt sich in die folgende Schritte unterteilen. Im ersten Schritt werden die Plättchen 6 auf die Patientenzähne 2 aufgeklebt. Im zweiten Schritt erfolgt eine optische dreidimensionale Aufnahme und wahlweise eine dreidimensionale Röntgenaufnahme der Patientenzähne mit den darauf geklebten Plättchen 6 aufgenommen wird. Anhand von Registrierungselementen mit Registrierungspunkten, die auf den Plättchen 6 angeordnet sind, werden die Positionen der Plättchen 6 relativ zu den Zähnen in der optischen dreidimensionalen Aufnahme identifiziert. Im nächsten Schritt erfolgt eine Planung des Bracketssystems 1, indem die Positionen der einzelnen Brackets 3 sowie der Verlauf des Bogens 4 festgelegt werden. Aus den festgelegten Positionen der virtuellen Brackets 3 und den Positionen der Plättchen 6 kann dann mittels der Planungseinheit berechnet werden an welcher Stelle die Klebeflächen der Brackets zu präparieren sind. Im letzten Schritt werden dann die präparierten Brackets 3 auf die Plättchen 6 aufgesetzt und auf die lingualen Zahnoberflächen geklebt.

Die Fig. 2a zeigt die linguale Zahnoberfläche 5 eines Schneidezahns 2, wobei das Plättchen 6 in etwa mittig im vorgesehen Klebebereich des Brackets aufgeklebt ist. Das Plättchen 6 weist Registrierungselemente 10 mit Registrierungspunkten 11 auf, die eine Form von runden Kugelabschnitten aufweisen und aus der Ebene des Plättchens herausragen. Sie sind besonders vorteilhaft für die Registrierung in einer dreidimensionalen optischen Aufnahme.

Die Figur 2b zeigt eine Skizze des Schneidezahns 2 aus der Figur 2a in einem weiteren Schritt des erfinderischen Verfahrens. Auf das Plättchen 6 mit Registrierungspunkten 11 ist ein präpariertes Bracket 3 aufgesetzt, das eine Aussparung 20 aufweist, die von der Form als Gegenstück exakt dem Plättchen 6 entspricht. Vor dem Aufkleben wird das Bracket 3 mit einer Klebeschicht beschichtet, die beim Aufkleben den gesamten Zwischenraum zwischen den Bracket 3 und der lingualen Zahnoberfläche 5 des Zahns 2 ausfüllt. Mittig durch das Bracket 3 verläuft der Bogen 4, der korrigierende Kräfte, wie Torsionskräfte, Zugkräfte und Druckkräfte auf den zu korrigierenden Schneidezahn 2 ausübt.

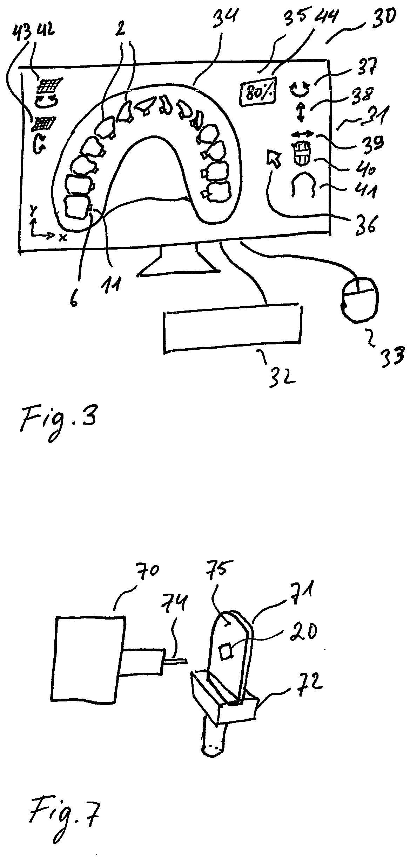

Die Figur 3 zeigt eine Ausführungsform der Planungseinheit 30, umfassend einen Bildschirm 31, eine Tastatur 32 und eine Maus 33. Der Bildschirm 31 zeigt ein Meistermodell 34, das aus den Bilddaten der dreidimensionalen optischen Aufnahme 50 in Kombination mit den Bilddaten einer dreidimensionalen Röntgenaufnahme 51 erstellt wurde. Zur Überlagerung der Bilddaten der dreidimensionalen optischen Aufnahme 50 und der Bilddaten der dreidimensionalen Röntgenaufnahme 51 wurden die Registrierungspunkte 11 der Plättchen 6 verwendet, die sowohl auf Grund der charakteristischen Form in der optischen dreidimensionalen Aufnahme als auch aufgrund ihrer Materialzusammensetzung mit einem höheren Absorptionskoeffizienten bezüglich Röntgenstrahlung in der dreidimensionalen Röntgenaufnahme registrierbar sind. Auf der virtuellen Arbeitsoberfläche 35 ist ein Cursor 36 zur virtuellen Bearbeitung des Meistermodells 34 dargestellt. Darüber hinaus sind mehrere virtuelle Werkzeuge dargestellt, nämlich ein erstes virtuelles Werkzeug 37 zur Drehung der virtuellen Zähne 2 um ihre Achse, die durch den Verlauf der Zahnwurzeln gegeben ist, ein zweites virtuelles Werkzeug 38 zur Verschiebung der virtuellen Zähne 2 entlang der Achse der Zähne 2, ein drittes virtuelles Werkzeug 39 zur seitlichen Verschiebung der virtuellen Zähne 2, um eventuelle Zahnlücken zu schließen, ein viertes virtuelles Werkzeug 40, das ein Modell eines Brackets 3 darstellt, welches für jeden Zahn individuell ausgewählt werden kann und ein fünftes virtuelles Werkzeug 41, das ein Modell des Bogens 4 darstellt. Zusätzlich sind Bedienungselemente 42 und 43 gezeigt, die das Rotieren des dreidimensionalen virtuellen Meistermodells 34 um die X- und Y-Achse erlauben. Das virtuelle Meistermodell 34 wird mittels der virtuellen Werkzeuge 37, 38, 39, 40 und 41 bearbeitet, um die Fehlstehlungen der Zähne des Patienten virtuell zu korrigieren und ein ideales virtuelles Setupmodell zu erstellen, bei dem alle Fehlstellungen der Zähne des Patienten korrigiert sind. Aus den Abweichungen des Meistermodells 34 und des korrigierten Setupmodells kann dann mittels der Planungseinheit berechnet werden, welche Positionsänderungen der einzelnen Zähne notwendig sind. Aus den korrigierten Positionsänderungen der einzelnen Zähne werden mittels der Planungseinheit die notwendigen korrigierenden Kräfte, die auf die Zähne einwirken sollen, ermittelt und zwar unter Berücksichtigung der Faktoren aus der optischen dreidimensionalen Aufnahme, nämlich die räumliche Anordnung und Form der Zähne, und unter Berücksichtung weiterer Faktoren aus der dreidimensionalen Röntgenaufnahme über eine Verteilung von harten und weichem Gewebe, nämlich die Position und die Abmessungen von Zahnwurzeln innerhalb einer apikalen Basis eines Kiefers, die Abmessungen des Kieferknochens, anatomische Grenzen, Form der Gingiva, Nasenbodens und die Position des Gaumens, wobei zur Berechnung die bekannten Materialkonstanten der verschiedenen Gewebearten und Materialien der Brackets 3 sowie des Bogens 4 verwendet werden. Im nächsten Schritt wird mittels der Planungseinheit berechnet, wie die Brackets 3 und der Bogen 4 anzuordnen sind, um diese notwendigen korrigierenden Kräfte auf die Patientenzähne 2 auszuüben. Eine Erfolgswahrscheinlichkeit 44 der gewünschten mechanischen dauerhaften Positionsänderungen der Patientenzähne 2 gemäß dem Setupmodell wird mittels der Planungseinheit 30 berechnet, wobei die bekannten Faktoren aus der optischen dreidimensionalen Aufnahme und der dreidimensionalen Röntgenaufnahme sowie die bekannten Materialkonstanten der verschiedenen Gewebearten und der Materialien verwendet werden. Falls die Erfolgswahrscheinlichkeit 44 zu gering ist, werden von der Planungseinheit 30 alternative Empfehlungen zur Behandlung, wie Extraktion, Strippen oder Operieren, vorgeschlagen. Zusätzlich zur Verbesserung der Planung können durch den Benutzer individuelle Behandlungsstrategien, nämlich ausgewählte Okklusionskonzepte, wie eine bioästhetische oder prothetische Versorgung, eine Berücksichtung der Vorgeschichte des jeweiligen Patienten und eine eventuelle notwendige Überkompensation der Korrektur, eingegeben werden und bei der Planung der Bracketsystems 1 berücksichtigt werden. Beispielsweise können bereits bekannte Implantatschrauben zur Verankerung von künstlichen Zähnen bezüglich ihrer Materialeigenschaften und ihre Lage bei der Planung berücksichtigt werden. Zur Verankerung von Zähnen, die in Ihrer Lage relativ zum Kiefer nicht korrigiert werden sollen, können Mini-Schrauben verwendet werden, die am Gaumen befestigt werden und somit die an den Brackets entstehenden korrigierenden Kräfte an den Gaumen ableiten. Der Vorteil dieser Mini-Schrauben ist, dass sie im Gegensatz zu Zähnen relativ zum Gaumen positionsstabil sind.

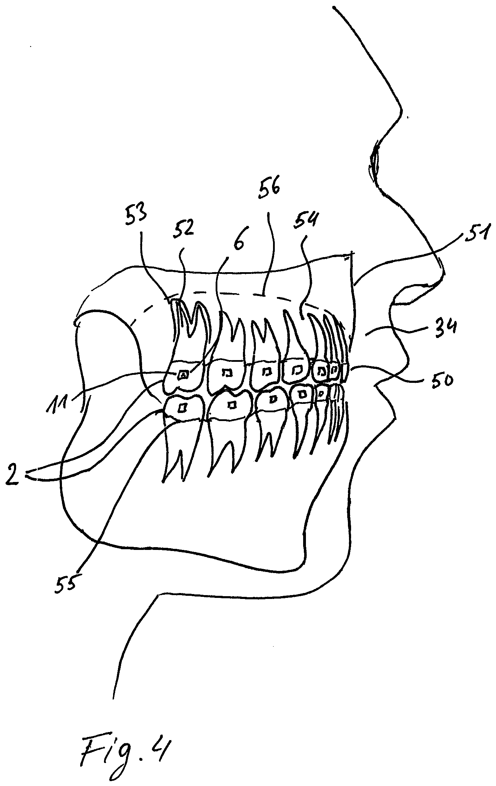

Die Figur 4 zeigt eine Skizze des Meistermodells 34 aus Figur 3, wobei die Bilddaten der dreidimensionalen optischen Aufnahme 50 durch Überlagerung von Registrierungspunkten 11 der Plättchen 6 mit den Bilddaten der dreidimensionalen Röntgenaufnahme 51 zusammengeführt sind. Aus dem virtuellen Meistermodell 54 können insbesondere Faktoren aus der optischen dreidimensionalen Aufnahme 50, wie die räumliche Anordnung und Form der Patientenzähne 2 sowie weitere Faktoren aus der dreidimensionalen Röntgenaufnahme 51 über die Verteilung von harten und weichem Gewebe ermittelt werden, nämlich die Positionen und die Abmessungen von Zahnwurzeln 52 innerhalb einer apikalen Basis 53, die Abmessungen des Kieferknochens 54, Form der Gingiva 55, weitere anatomische Grenzen, der Nasenboden und die Position des Gaumens 56. Dabei ist die Lage des Nasenbodens wichtig für die Korrektur der von Frontzähne Die Figur 4 stellt eine seitliche Ansicht des virtuellen Meistermodells dar, das mittels der Planungseinheit 30 am Bildschirm 31 nach Figur 3 dargestellt ist.

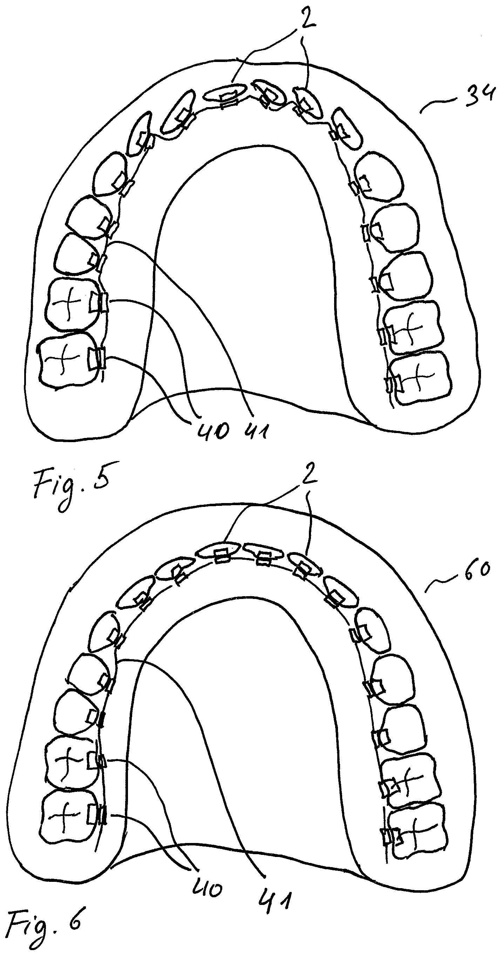

Die Figur 5 zeigt das virtuelle Meistermodell 34 nach Figur 3 und nach Figur 4 mit den Fehlstellungen der Patientenzähne 2. Die einzelne Zähne 2 werden, wie in Figur 3 dargestellt, mittels der Planungseinheit 30 unter Verwendung der virtuellen Werkzeuge 37, 38, 39, 40 und 41 virtuell korrigiert und damit ein korrigiertes ideales Setupmodell erstellt.

Die Figur 6 zeigt das korrigierte ideale Setupmodell 60, das mittels der Planungseinheit nach Figur 3 aus dem virtuellen Meistermodell 34 nach Figur 5 durch den Benutzer virtuell erstellt wurde. Dabei wurden alle Fehlstellungen der Zähne 2 beseitigt, indem die Zähne mittels des virtuellen Werkzeugs 37 um die eigene Achse gedreht wurden, mittels des virtuellen Werkzeugs 38 in ihre Höhe verändert wurden und mittels der virtuellen Werkzeugs 39 seitlich verschoben wurden, um eventuelle Zahnlücken zu schließen. Aus den notwendigen Positionsänderungen der einzelnen Zähne 2, die sich aus dem Vergleich des Meistermodells 34 und des Setupmodells 60 ergeben, werden mittels der Planungseinheit 30 die notwendigen korrigierende Kräfte ermittelt und dementsprechend die Modelle 40 der Brackets 3 und das Modell 41 des Bogens 4 geplant. Durch den Benutzer können zusätzlich individuelle Behandlungsstrategien, wie die bioästhetische Versorgung, die Vorgeschichte des jeweiligen Patienten und eine eventuell notwendige Überkompensation der Korrektur, eingegeben werden und bei der Planung der Modelle 40 der Brackets und des Modells 41 des Bogens 4 berücksichtigt werden. Aus der bekannten Position der aufgeklebten Plättchen 6 auf den Zähnen 2 und der geplanten Anordnung der Modelle 40 der Brackets 3 wird mittels der Planungseinheit 30 ermittelt, an welcher Stelle die unpräparierten Brackets mit einer zum Plättchen passenden Aussparung auf der Klebefläche mittels einer CAD/CAM-Einheit präpariert werden müssen.

Die Figur 7 zeigt eine Skizze einer CAD/CAM-Einheit 70, die zur Anpassung eines nicht präparierten Brackets 71 verwendet wird. Das nicht präparierte Bracket wird auf einem Halter fixiert und eine mittels der Planungseinheit 3 nach Figur 3 geplante Aussparung 20 mit einem Fräswerkzeug 74 wird herausgefräst. Zusätzlich wird die gesamte Klebefläche 75 des nicht präparierten vorgefertigten Brackets 71 mit dem Fräswerkzeug 74 so bearbeitet, dass die Klebefläche ein Gegenstück zur Form der jeweiligen Zahnoberfläche 5 bildet, die mit diesem Bracket 71 beklebt werden soll. Damit wird eine präzise Passung der Brackets 3 an die Zähne 2 erreicht.

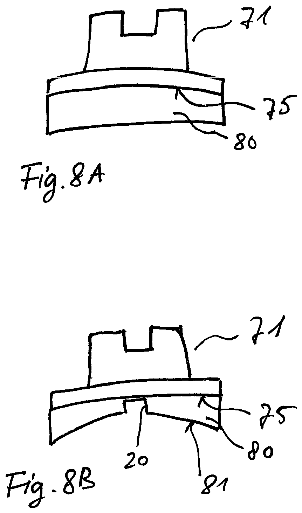

Die Figur 8A beschreibt eine alternative Ausführungsform eines zu präparierenden Brackets 71, wobei die Klebefläche 75 mit einer Kunststoffschicht 80 beschichtet wird.

Die Figur 8B zeigt das Bracket 71 aus Figur 8A nach der Bearbeitung mit einem Fräswerkzeug 74, wie in Figur 7 dargestellt. Die Kunststoffschicht 80 wird mit dem Fräswerkzeug 74 aus Figur 7 so bearbeitet, dass die bearbeitete Oberfläche 81 der Kunststoffschicht 80 ein Gegenstück zur Form der jeweiligen Zahnoberfläche 5 bildet. Zusätzlich wird eine Aussparung 20 zur präzisen Passung an das jeweilige Plättchen 6 aus der Kunststoffschicht 80 herausgearbeitet. Dadurch weist das Werkzeug 74 eine längere Nutzungsdauer auf als bei einer Bearbeitung von Metall, wie in Figur 7.

### Bezugszeichenliste

- 1: Bracketsystem
- 2: Patientenzähne
- 3: Bracket
- 4: Bogen
- 5: Zahnoberfläche
- 6: Plättchen
- 10: Registrierungselemente
- 11: Registrierungspunkte
- 20: Aussparung
- 30: Planungseinheit
- 31: Bildschirm
- 32: Tastatur
- 33: Maus
- 34: Meistermodell
- 35: virtuelle Arbeitsoberfläche
- 36: Cursor
- 37: erstes Werkzeug
- 38: zweites Werkzeug
- 39: drittes Werkzeug
- 40: viertes Werkzeug
- 41: fünftes Werkzeug
- 44: Erfolgswahrscheinlichkeit
- 46: virtueller Zahn
- 50: dreidimensionale optische Aufnahme
- 51: dreidimensionale Röntgenaufnahme
- 52: Zahnwurzel
- 53: apikale Basis
- 54: Kieferknochen
- 55: Gingiva
- 56: Position des Gaumens
- 60: Setupmodell
- 70: CAD/CAM-Einheit
- 71: nicht präpariertes Bracket
- 73: Aussparung
- 74: Fräswerkzeug
- 75: Klebefläche

## Patentansprüche

1. Verfahren zur Planung eines Bracketsystems (1) zur Korrektur von Fehlstellungen von Zähnen (2), bestehend aus mehreren Brackets (3) und einem Bogen (4), **dadurch gekennzeichnet, dass** auf bestimmten Zahnoberflächen (5) der zu korrigierenden Zähne (2) pro Zahn jeweils mindestens ein Plättchen (6) bereits angebracht sind, wobei eine dreidimensionale optische Aufnahme (50) des zu behandelnden Zahnbereichs aufgenommen wird, wobei die Plättchen (6) Registrierungselemente (10) mit Registrierungspunkten (11) aufweisen, wobei die Registrierungspunkte (11) eine charakteristische Form zur Registrierung in der dreidimensionalen optischen Aufnahme (50) aufweisen, wobei die vorgefertigten Brackets (6) mittels einer CAD/CAM-Einheit (70) an das aufgeklebte Plättchen (6) angepasst werden und die gesamte Klebefläche (75) jedes Brackets (3) als Gegenstück entsprechend der Form der jeweiligen Zahnoberfläche (5) geformt wird, um eine einwandfreie Passung zu ermöglichen.

2. Verfahren nach Anspruch 1, **dadurch gekennzeichnet, dass**, zusätzlich zur dreidimensionalen optischen Aufnahme (50) eine dreidimensionale Röntgenaufnahme (51) des zu behandelnden Zahnbereichs aufgenommen wird, wobei die Registrierungselemente (10) mit Registrierungspunkten (11) der Plättchen (6) derart aufgebaut sind, dass sie zur Registrierung in der dreidimensionalen Röntgenaufnahme (51) bezüglich Röntgenstrahlung sensitiv sind, indem die Registrierungspunkte (11) aus einem Material aufgebaut sind, der einen Absorptionskoeffizienten bezüglich Röntgenstrahlung aufweist, der sich deutlich vom Absorptionskoeffizienten des übrigen Materials der Plättchen (6) unterscheidet.

3. Verfahren nach Anspruch 2, **dadurch gekennzeichnet, dass** mittels einer Planungseinheit (30) zur Planung des Bracketsystems (1) computergestützt die Bilddaten der dreidimensionalen optischen Aufnahme (50) und die Bilddaten der dreidimensionalen Röntgenaufnahme (51) durch Überlagerung von Registrierungspunkten (11) der Registrierungselemente (10) in der dreidimensionalen optischen Aufnahme (50) und der dreidimensionalen Röntgenaufnahme (51) zusammengeführt werden, so dass eine eindeutige Lagebeziehung der Plättchen (6) zur optischen dreidimensionalen Aufnahmen (50) und zur dreidimensionalen Röntgenaufnahme (51) festgestellt wird.

4. Verfahren nach einem der Ansprüche 1 bis 3, **dadurch gekennzeichnet, dass** mittels der Planungseinheit (30) eine computergestützte Planung des anzufertigenden Bracketsystems (1) anhand der überlagerten dreidimensionalen optischen Aufnahme (50) und der dreidimensionalen Röntgenaufnahme (51) erfolgt.

5. Verfahren nach einem der Ansprüche 1 bis 4, **dadurch gekennzeichnet, dass** anhand der dreidimensionalen optischen Aufnahme (50) ein virtuelles Meistermodell (34) des Oberkiefers und/oder Unterkiefers erstellt wird, das die Fehlstellungen der Zähne des Patienten beinhaltet, wobei durch den Benutzer mittels der Planungseinheit (30) die einzelnen Zähne virtuell korrigiert werden und ein ideales virtuelles Setupmodell (57) erstellt wird, bei dem alle Fehlstellungen der Zähne des Patienten korrigiert sind, wobei aus den Abweichungen des Meistermodells (34) und des Setupmodells (57) korrigierenden Positionsänderungen der einzelnen Zähne berechnet werden, wobei aus den korrigierenden Positionsänderungen die Anordnung der Brackets (3) und des Bogens (5) des Bracketsystems (1) bestimmt wird.

6. Verfahren nach einem der Ansprüche 1 bis 5, **dadurch gekennzeichnet, dass** bei der Planung des Bracketsystems (1) Faktoren aus der optischen dreidimensionalen Aufnahmen (50), nämlich die räumliche Anordnung und Form der Zähne (2), und weitere Faktoren aus der dreidimensionalen Röntgenaufnahme (51) über eine Verteilung von hartem und weichem Gewebe, nämlich die Position und die Abmessungen von Zahnwurzeln (52) innerhalb einer apikalen Basis (53) eines Kiefers des Patienten, die Abmessungen des Kieferknochens (54), Form der Gingiva (55) anatomische Grenzen, Nasenboden und/oder die Position des Gaumens (56), berücksichtigt werden, indem aus der Anordnung des harten und weichen Gewebes unter Heranziehung der bekannten Materialkonstanten der verschiedenen Gewebearten eine Erfolgswahrscheinlichkeit (44) der gewünschten mechanischen dauerhaften Positionsänderung der Zähne nach dem Setupmodell (57) berechnet wird.

7. Verfahren nach Anspruch 6, **dadurch gekennzeichnet, dass** falls die von der Planungseinheit (30) berechnete Erfolgswahrscheinlichkeit (44) gering ist, mittels der Planungseinheit (30) alternative Empfehlungen zur Behandlung, wie Extraktion, Strippen oder Operieren, vorgeschlagen werden.

8. Verfahren nach einem der Ansprüche 1 bis 7, **dadurch gekennzeichnet, dass** zusätzlich durch den Benutzer individuelle Behandlungsstrategien, nämlich ein bestimmtes Okklusionskonzept, wie eine bioästhetische Versorgung, eine Berücksichtigung der Vorgeschichte des jeweiligen Patienten und/oder eine notwendige Überkompensation der Korrektur, eingegeben werden und bei der Planung des Bracketsystems (1) von der Planungseinheit (30) beachtet werden.

9. Verfahren nach einem der Ansprüche 1 bis 8, **dadurch gekennzeichnet, dass** nach der Planung des Bracketsystems (1) mittels der Planungseinheit (30) vorgefertigte Brackets (3) mittels einer CAD/CAM-Einheit (70) an die aufgeklebten Plättchen (6) angepasst werden, indem auf einer Klebefläche (75) jedes Brackets (3) eine Aussparung (20) herausgearbeitet wird, die als Gegenstück der Form des Plättchens (6) entspricht, so dass beim Befestigen des Brackets (3) an dem Plättchen (6) die geplante Position relativ zum Zahn (46) erreicht wird.

10. Bracketsytem (1) zur Korrektur von Fehlstellungen, von Zähnen (2), bestehend aus mehreren Brackets (3), einen Bogen (4) und mehreren Plättchen (6), **dadurch gekennzeichnet, dass** die Plättchen (6) eine Klebefläche zum Kleben auf eine bestimmte Zahnoberfläche (5) eines zu korrigierenden Zahns (2) und Registrierungselemente (10) mit Registrierungspunkten (11) aufweisen, die eine charakteristische Form zur Registrierung in einer dreidimensionalen optischen Aufnahme (50) ermöglichen, wobei die Brackets (3) eine Aussparung (20) aufweisen, die als Gegenstück zu der Form der Plättchen (6) geformt ist, um eine genaue Passung des Plättchen (6) an das jeweilige Bracket (3) zu gewährleisten.

11. Bracketsystem nach Anspruch 10, **dadurch gekennzeichnet, dass**, die Registrierungselemente (10) mit Registrierungspunkten (11) bezüglich Röntgenstrahlung sensitiv sind, wobei die Registrierungspunkte (11) aus einem Material aufgebaut sind, der einen Absorptionskoeffizienten bezüglich Röntgenstrahlung aufweist, der sich deutlich vom Absorptionskoeffizienten des übrigen Materials der Plättchen (6) unterscheidet, um die Registrierung in einer dreidimensionalen Röntgenaufnahme (51) zu ermöglichen.

12. Bracketsystem nach Anspruch 10 oder 11, **dadurch gekennzeichnet, dass** die Brackets (3) eine Klebefläche (75) aufweisen, die jeweils als Gegenstück zu der zu beklebenden Zahnoberfläche (5) der Zähne (2) geformt ist, um eine bessere Passung der Klebung zu erreichen.

## Claims

1. Method for planning a bracket system (1) for correction of incorrect positions of teeth (2), consisting of multiple brackets (3) and an arch (4), **characterized in that** respectively at least one plate (6) has already been attached per tooth to specific tooth surfaces (5) of the teeth (2) to be corrected, wherein a three-dimensional optical exposure (50) of the dental zone to be treated is made, wherein the plates (6) have registration elements (10) with registration points (11), wherein the registration points (11) exhibit a characteristic shape for registration in the three-dimensional optical exposure (50), wherein the prefabricated brackets (6) are adapted to the adhered plates (6) by means of a CAD/CAM unit (70), and the entire adhesive surface (75) of each bracket (3) is shaped as a counterpart corresponding to the shape of the respective tooth surface (5) in order to enable an unproblematic fit.

2. Method according to claim 1, **characterized in that** a three-dimensional x-ray exposure (51) of the dental zone to be treated is made in addition to the three-dimensional optical exposure (50), wherein the registration elements (10) with registration points (11) of the plates (6) are designed such that they are sensitive to x-ray radiation for registration in the three-dimensional x-ray exposure (51), **in that** the registration points (11) are made of a material that has an absorption coefficient with regard to x-ray radiation that differs markedly from the absorption coefficient of the remaining material of the plate (6).

3. Method according to claim 2, **characterized in that** the image data of the three-dimensional optical exposure (50) and the image data of the three-dimensional x-ray exposure (51) are merged via superposition of registration points (11) of the registration elements (10) in the three-dimensional optical exposure (50) and the three-dimensional x-ray exposure (51) in a computer-aided manner by means of a planning unit (30) for planning the bracket system (1), such that a clear positional relationship of the plates (6) to the optical three-dimensional exposures (50) and to the three-dimensional x-ray exposure (51) is established.

4. Method according to one of claims 1 through 3, **characterized in that** a computer-aided planning of the bracket system (1) to be prepared takes place by means of the planning unit (30), using the superimposed three-dimensional optical exposure (50) and the three-dimensional x-ray exposure (51).

5. Method according to one of claims 1 through 4, **characterized in that** a virtual master model (34) of the maxilla and/or mandible that includes the incorrect positions of the teeth of the patient is created using the three-dimensional optical exposure (50), wherein the individual teeth are virtually corrected by the user by means of the planning unit (30), and an ideal virtual setup model (57) is created in which all incorrect positions of the teeth of the patient are corrected, wherein corrective position changes of the individual teeth are calculated from the deviations of the master model (34) and the setup model (57), wherein the arrangement of the brackets (3) and of the arch (5) of the bracket system (1) is determined from the corrective position changes.

6. Method according to one of claims 1 through 5, **characterized in that** factors from the optical three-dimensional exposures (50), viz., the spatial arrangement and shape of the teeth (2), and additional factors from the three-dimensional x-ray exposure (51) regarding a distribution of hard and soft tissue, viz., the position and the dimensions of tooth roots (52) within an apical base (53) of a jaw of the patient, the dimensions of the jawbone (54), shape of the gingiva (55), anatomical boundaries, nasal floor, and/or the position of the palate (56), are taken into account in the planning of the bracket system (1), **in that** a probability of success (44) of the desired mechanical, long-term position change of the teeth according to the setup model (57) is calculated from the arrangement of the hard and soft tissue, with consideration of the known material constants of the various tissue types.

7. Method according to claim 6, **characterized in that** alternative recommendations for treatment, such as extraction, stripping, or operating, are proposed by means of the planning unit (30) in the event that the probability of success (44) calculated by the planning unit (30) is low.

8. Method according to one of claims 1 through 7, **characterized in that** individual treatment strategies, viz., a specific occlusion design such as a bio-aesthetic accommodation, a consideration of the anamnesis of the respective patient, and/or a necessary overcompensation of the correction, are additionally input by the user and are heeded in the planning of the bracket system (1) by the planning unit (30).

9. Method according to one of claims 1 through 8, **characterized in that** brackets (3) prefabricated after the planning of the bracket system (1) by means of the planning unit (30) are adapted to the adhered plates (6) by means of a CAD/CAM unit (70), **in that** a relief (20) is carved out on the adhesive surface (75) of each bracket (3), which relief (20) corresponds as a counterpart to the shape of the plate (6), so that the planned position relative to the tooth (46) is achieved upon attachment of the bracket (3) to the plate (6).

10. Bracket system (1) for correction of incorrect positions of teeth (2), consisting of multiple brackets (3), an arch (4), and multiple plates (6), **characterized in that** the plates (6) have an adhesive surface for adhesion to a specific tooth surface (5) of a tooth (2) to be corrected and registration elements (10) having registration points (11) that enable a characteristic shape for registration in a three-dimensional optical exposure (50), wherein the brackets (3) have a relief (20) that is shaped as a counterpart to the shape of the plate (6) in order to ensure a precise fit of the plate (6) to the respective bracket (3).

11. Bracket system according to claim 10, **characterized in that** the registration elements (10) with registration points (11) are sensitive to x-ray radiation, wherein the registration points (11) are made from a material that has an absorption coefficient with regard to x-ray radiation that differs markedly from the absorption coefficients of the remaining material of the plate (6), in order to enable the registration in a three-dimensional x-ray exposure (51).

12. Bracket system according to claim 10 or 11, **characterized in that** the brackets (3) have an adhesive surface (75) that is respectively shaped as a counterpart to the tooth surface (5) of the teeth (2) in order to achieve a better fit of the adhesion.

## Revendications

1. Procédé de planification d'un système de brackets (1) pour la correction d'anomalies de position de dents (2), constitué de plusieurs brackets (3) et d'un arc (4), **caractérisé en ce qu'**au moins une plaquette (6) par dent est déjà appliquée respectivement sur des surfaces de dents (5) données des dents à corriger (2), un cliché optique en trois dimensions (50) de la zone de dent à traiter étant enregistré, les plaquettes (6) présentant des éléments d'enregistrement (10) pourvus de points d'enregistrement (11), les points d'enregistrement (11) présentant une forme caractéristique pour l'enregistrement dans le cliché optique en trois dimensions (50), les brackets fabriqués au préalable (6) étant adaptés au moyen d'une unité CAO/FAO (70) à la plaquette collée (6) et la totalité de la surface adhésive (75) de chaque bracket (3) étant formée comme contrepartie correspondant à la forme de la surface de dent (5) respective, afin de permettre un ajustement impeccable.

2. Procédé selon la revendication 1, **caractérisé en ce que**, en plus du cliché optique en trois dimensions (50), une radiographie en trois dimensions (51) de la zone de dent à traiter est enregistrée, les éléments d'enregistrement (10) pourvus de points d'enregistrement (11) des plaquettes (6) étant conçus de sorte qu'ils soient sensibles au rayons X pour l'enregistrement dans la radiographie en trois dimensions (51), **en ce que** les points d'enregistrement (11) sont conçus en un matériau, qui présente un coefficient d'absorption des rayons X, qui se distingue sensiblement du coefficient d'absorption du reste du matériau des plaquettes (6).

3. Procédé selon la revendication 2, **caractérisé en ce que**, au moyen d'une unité de planification (30) pour la planification du système de brackets (1), les données d'image du cliché optique en trois dimensions (50) et les données d'image de la radiographie en trois dimensions (51) sont fusionnées de manière assistée par ordinateur par superposition des points d'enregistrement (11) des éléments d'enregistrement (10) dans le cliché optique en trois dimensions (50) et dans la radiographie en trois dimensions (51), de sorte qu'une relation de position unique des plaquettes (6) soit déterminée par rapport au cliché optique en trois dimensions (50) et à la radiographie en trois dimensions (51).

4. Procédé selon l'une quelconque des revendications 1 à 3, **caractérisé en ce que**, au moyen de l'unité de planification (30), une planification assistée par ordinateur du système de brackets à fabriquer (1) est réalisée à l'aide du cliché optique en trois dimensions (50) et de la radiographie en trois dimensions (51).

5. Procédé selon l'une quelconque des revendications 1 à 4, **caractérisé en ce que**, à l'aide du cliché optique en trois dimensions (50), un modèle maître virtuel (34) de la mâchoire supérieure et/ou de la mâchoire inférieure est réalisé, qui contient les anomalies de position des dents du patient, les dents individuelles étant corrigées virtuellement par l'utilisateur au moyen de l'unité de planification (30) et un modèle de configuration virtuel idéal (57) étant réalisé, dans lequel toutes les anomalies de position des dents du patient sont corrigées, des modifications de position correctives des dents individuelles étant calculées à partir des écarts du modèle maître (34) et du modèle de configuration (57), l'agencement des brackets (3) et de l'arc (5) du système de brackets (1) étant déterminé à partir des modifications de position correctives.

6. Procédé selon l'une quelconque des revendications 1 à 5, **caractérisé en ce que**, lors de la planification du système de brackets (1), des facteurs du cliché optique en trois dimensions (50), à savoir l'agencement spatial et la forme des dents (2), ainsi que d'autres facteurs de la radiographie en trois dimensions (51) concernant une répartition des tissus durs et mous, à savoir la position et les dimensions des racines dentaires (52) au sein d'une zone apicale (53) d'une mâchoire du patient, les dimensions de l'os maxillaire (54), la forme des gencives (55), les limites anatomiques, le plancher du nez et/ou la position du palais (56), sont pris en considération, **en ce qu'**on calcule, à partir de l'agencement du tissu dur et du tissu mou, tout en tenant compte des constantes de matériau connues des différents types de tissus, une probabilité de réussite (44) de la modification de position mécanique durable souhaitée des dents selon le modèle de configuration (57).

7. Procédé selon la revendication 6, **caractérisé en ce que**, au cas où la probabilité de réussite (44) calculée par l'unité de planification (30) est faible, des recommandations alternatives de traitement, comme l'extraction, le limage ou l'opération, sont proposées par l'unité de planification (30).

8. Procédé selon l'une quelconque des revendications 1 à 7, **caractérisé en ce que** l'utilisateur saisit, en plus, des stratégies de traitement individuelles, à savoir un concept d'occlusion déterminé, comme un soin bio-esthétique, une prise en considération des antécédents du patient individuel et/ou une surcompensation nécessaire de la correction et celles-ci sont prises en compte lors de la planification du système de brackets (1) par l'unité de planification (30).

9. Procédé selon l'une quelconque des revendications 1 à 8, **caractérisé en ce que**, après la planification du système de brackets (1) au moyen de l'unité de planification (30), les brackets fabriqués au préalable (3) sont adaptés au moyen d'une unité CAO/FAO (70) aux plaquettes collées (6), **en ce que**, sur une surface adhésive (75) de chaque bracket (3), un évidement (20) est façonné, qui correspond, en tant que contrepartie, à la forme de la plaquette (6) de sorte que lors de la fixation du bracket (3) sur la plaquette (6), la position planifiée par rapport à la dent (46) est atteinte.

10. Système de brackets (1) pour la correction d'anomalies de position de dents (2), constitué de plusieurs brackets (3), d'un arc (4) et de plusieurs plaquettes (6), **caractérisé en ce que** les plaquettes (6) présentent une surface adhésive à coller sur une surface de dent déterminée (5) d'une dent à corriger (2) et des éléments d'enregistrement (10) pourvus de points d'enregistrement (11), qui présentent une forme caractéristique pour permettre l'enregistrement dans un cliché optique en trois dimensions (50), les brackets (3) présentant un évidement (20), qui est formé comme une contrepartie à la forme des plaquettes (6), afin de garantir un ajustement précis de la plaquette (6) au bracket respectif (3).

11. Système de brackets selon la revendication 10, **caractérisé en ce que** les éléments d'enregistrement (10) pourvus de points d'enregistrement (11) sont sensibles aux rayons X, les points d'enregistrement (11) étant conçus en un matériau, qui présente un coefficient d'absorption des rayons X, qui se distingue nettement du coefficient d'absorption du reste du matériau des plaquettes (6), afin de permettre l'enregistrement dans une radiographie en trois dimensions (51).

12. Système de brackets selon la revendication 10 ou 11, **caractérisé en ce que** les brackets (3) présentent une surface adhésive (75), qui est formée respectivement comme une contrepartie à la surface de dent à coller (5) des dents (2), afin d'obtenir un meilleur ajustement du collage.
